# EUROPÄISCHE PATENTSCHRIFT

(11) **EP 0 968 420 B1**
(45) Veröffentlichungstag und Bekanntmachung des Hinweises auf die Patenterteilung: **11.09.2002**
(21) Anmeldenummer: 98966212.7
(22) Anmeldetag: 15.12.1998
(51) Int. Cl.: G01N 33/24, G01N 7/14, G01N 25/56

(54) **VERFAHREN ZUM MESSEN VON SORBATGEHALTEN IN SUBSTRATEN**
METHODS FOR MEASURING THE SORBATE CONTENT OF SUBSTRATES
PROCEDES POUR MESURER DES TENEURS EN SORBAT DANS DES SUBSTRATS

(30) Priorität: 17.12.1997 DE 19756170
(43) Veröffentlichungstag der Anmeldung: 05.01.2000
(73) Patentinhaber: LPKF Laser & Electronics AG, D-30827 Garbsen/OT Berenbostel (DE)
(72) Erfinder: KICKELHAIN, Jörg, D-31535 Neustadt (DE); SCHULZ, Olaf, D-13086 Berlin (DE); IMKENBERG, Frank, D-30167 Hannover (DE)
(74) Vertreter: Braun, Dieter, Dipl.-Ing.
(86) Internationale Anmeldenummer: DE9803691
(87) Internationale Veröffentlichungsnummer: WO99031502

(56) Entgegenhaltungen:
- DE-A- 3 819 335
- DE-A- 4 334 435
- US-A- 3 739 629
- US-A- 4 215 568
- BALASCIO CARMINE C ET AL: "Comparative study of moisture sensors for use in mushroom beds" TRANS ASAE MAY-JUN 1989, Bd. 32, Nr. 3, Mai 1989, Seiten 928-933, XP002102904

## Beschreibung

Die Erfindung betrifft ein Verfahren zum Messen von Sorbatgehalten in Substraten, insbesondere von Wassergehalten in Kompost, und ferner einen Sensor zur Durchführung des Verfahrens.

Sorbate, also bei einer Sorption aufgenommene Stoffe, insbesondere Wasser, stellen bei der Herstellung, der Bearbeitung, dem Transport und der Lagerung von Substraten einen wichtigen Faktor dar. So lassen sich über die Einstellung der Feuchte des Substrats Bearbeitungseigenschaften, Gewicht, Volumen und mögliche ablaufende biologische Prozesse oft stark beeinflussen. Daher ist zum Beispiel eine exakte Bestimmung der Feuchte des Substrats, wie auch der Umgebung, von großer Bedeutung.

Anders als bei der Messung von Sorbaten in der Umgebung steht für die Bestimmung des Sorbatgehaltes von Feststoffen nur eine relativ geringe Anzahl von Meßverfahren zur Verfügung.

Die Methode der gravimetrischen Bestimmung des Wassergehalts eines Substrats ist z. B. Grundlage der in der DE 38 19 335 A1 beschriebene Vorrichtung bzw. des dazugehörigen Verfahrens. Bei dieser Methode wird durch Entnahme von Proben und anschließende Trocknung bzw. thermisches Austreiben der flüchtigen Bestandteile aus der Probe und durch Sammeln des austretenden Wassers dessen Masse bestimmt. Diese Methode weist den Nachteil auf, daß mit ihr keine Ergebnisse für eine gewünschte vorausschauende Prozeßführung gewonnen werden können, die beispielsweise für einen wirtschaftlichen Betrieb technisch aufwendiger Kompostierungsanlagen unbedingt erforderlich ist. Ein weiterer Nachteil des gravimetrischen Meßverfahrens ist, daß nicht nur der Feuchtegehalt des Substrats, der durch eine physikochemische oder eine mechanische Sorption bzw. Bindung (siehe z. B. A. W. Lykow: "Experimentelle und theoretische Grundlagen der Trocknung", VEB Verlag Technik Berlin, S. 62-63) bewirkt ist und biologisch relevant ist, gemessen wird, sondern auch das chemisch gebundene Wasser, das sehr fest gebunden und daher biologisch nicht relevant ist, Eingang in die Messung findet.

Bekannte Feuchtesensoren stellen sich als zu ungenau bzw. als nicht anwendbar heraus.

Aus der Lebensmittelindustrie ist ein tensiometrisches Meßverfahren bekannt, bei dem ein eine Meßkammer aufweisender Sensor beispielsweise auf einer Meßlanze in das Substrat eingeführt wird (W. Lück: "Feuchtigkeit - Grundlagen, Messen, Regeln", R. Oldenbourg, München, Wien, 1964, S. 179 - 182). Bei der Feuchtemessung findet aufgrund von Diffusion von Wassermolekülen ein Ausgleich zwischen dem Wasserdampfpartialdruck des Substrats und dem Wasserdampfpartialdruck der Luft als Trägermedium in der Meßkammer statt. Nach einer gewissen Zeit ist somit die Feuchte der Luft in der Meßkammer konstant. Bei dieser Feuchte, der sogenannten Gleichgewichtsfeuchte, ist die Luft in der Meßkammer im Gleichgewicht, d. h. es verlassen das Substrat genauso viele Wassermoleküle, wie in das Substrat hineingehen.

Diese Gleichgewichtsfeuchte stellt einen bestimmten Wert der relativen Luftfeuchte (Verhältnis von absoluter zu maximaler Luftfeuchte) dar, von dem auf den Feuchtegehalt des Substrats zurückgeschlossen werden kann, wenn die Temperatur des Substrats bekannt ist.

Dieses auf der Nutzung der Eigenschaften der Gleichgewichtsfeuchte beruhende Verfahren gibt es in verschiedenen Ausgestaltungsformen, so z. B. auch unter Verwendung eines Oberflächensensors für flächige Stoffe. Ein solches System wird u. a. in der DE 36 34 518 A1 beschrieben.

Das bekannte tensiometrische Verfahren hat gegenüber dem gravimetrischen Verfahren den Vorteil, daß das chemisch gebundene Wasser keinen Eingang in die Messung findet. Vielmehr wird nur der Feuchtegehalt des Substrats gemessen, der biologisch relevant ist. Ein Nachteil des Verfahrens zum Messen der Feuchte mittels der Gleichgewichtsfeuchte ist jedoch, daß es nur bei hygroskopisch feuchten Stoffen anwendbar ist, nicht jedoch bei nassen Stoffen, da über letzteren die Luft wasserdampfgesättigt ist (siehe W. Lück, a. a. O.). So konnte das bekannte Verfahren bisher nur in Bereichen eingesetzt werden, in denen relativ geringe Feuchten zu bestimmen bzw. zu kontrollieren waren. So kann dieses Meßverfahren z. B. nur bei Müllkompost bis zu einem Feuchtegehalt des Substrats von etwa 40 % angewendet werden. Bei Biokompost sind Messungen nur bis ca. 10 % Feuchtegehalt des Substrats möglich. Dies ist darin begründet, daß oberhalb dieses Substratfeuchtegehaltes die Luft in der Meßkammer in der Regel gesättigt ist, also die relative Luftfeuchte 100 % beträgt. Bei einer Sättigung der Luft ist jedoch kein Rückschluß auf den Feuchtegehalt des Substrats möglich. Bei der Kompostierung von biogenen Abfällen ist aber ein Feuchtebereich des Komposts zwischen 50 und 60 % interessant, da die Mikroorganismen bei diesen Umgebungsbedingungen optimal arbeiten.

Andererseits ist es jedoch wünschenswert, die Bestimmung des Wassergehalts des Substrats über die Messung der Gleichgewichtsfeuchte vorzunehmen, um das chemisch gebundene Wasser nicht mit in die Messung einzubeziehen.

Der Erfindung liegt daher die Aufgabe zugrunde, ein Verfahren zur Messung von Sorbatgehalten in Substraten zur Verfügung zu stellen, das auf der Messung der Gleichgewichtsfeuchte basiert und auch eine Messung eines relativ hohen Sorbatgehalts des Substrats ermöglicht, und ferner einen Sensor zur Durchführung des Verfahrens zur Verfügung zu stellen, mit dem die Beeinflussung des Substrats durch die Messung gering ist.

Diese Aufgabe wird in bezug auf das Verfahren durch die Merkmale des Anspruchs 1 gelöst.

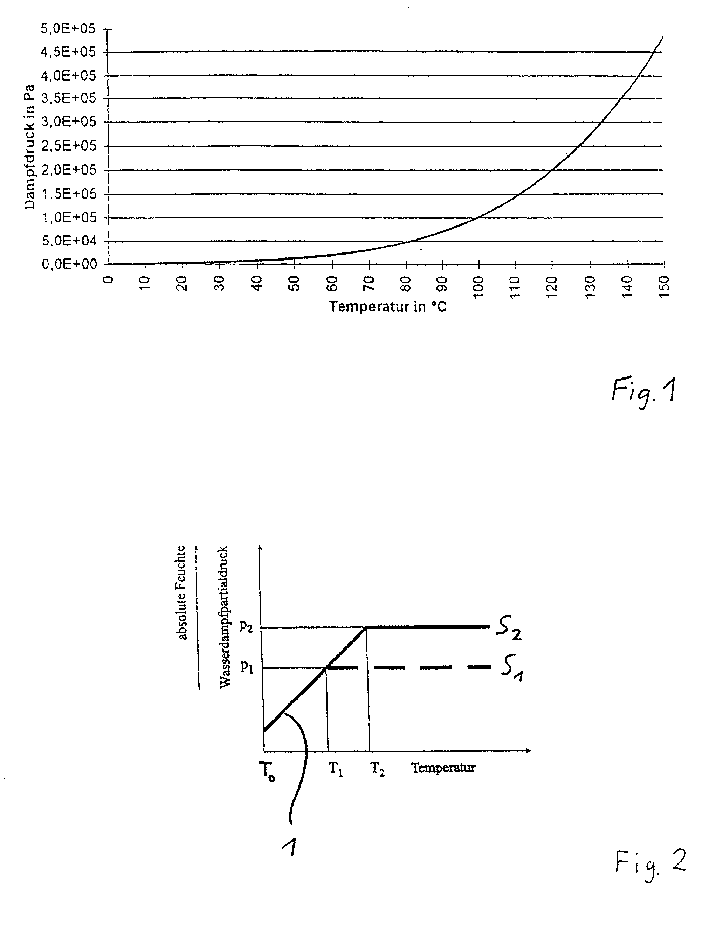

Die Erfindung nutzt das physikalische Prinzip aus, daß die Aufnahmefähigkeit eines Mediums für ein Sorbat sich mit der Temperatur ändert. So tritt beispielsweise eine Sättigung von Luft mit Wassermolekülen um so später ein, je höher die Temperatur der Luft ist. Zur Veranschaulichung ist in Figur 1 der maximale Wasserdampfdruck von Luft in Abhängigkeit von der Temperatur der Luft dargestellt.

Bei dem erfindungsgemäßen Verfahren wird ein Sensor mit einer Meßkammer verwendet, die ein Trägermedium enthält, dessen Sorbatpartialdruck bestimmt wird, aus dem unter Berücksichtigung der gemessenen, im wesentlichen konstanten Temperatur des Substrats der Sorbatgehalt des Substrats ermittelt wird, Dadurch, daß die Temperatur des Trägermediums in der Meßkammer bis zu einem Wert und etwas über diesen hinaus variiert wird, bei welchem sich der Sorbatpartialdruck in der Meßkammer nicht mehr in Abhängigkeit von der Temperatur des Trägermediums ändert, wird erreicht, daß eine Temperatur eingestellt wird, bei der das Trägermedium in der Meßkammer nicht mit Sorbatmolekülen gesättigt ist. Aus diesem Sorbatpartialdruck kann somit auf den Sorbatgehalt des Substrats zurückgeschlossen werden. Denn der von der Temperatur des Trägermediums unabhängige Sorbatpartialdruck des Trägermediums ist gleich dem Dampf- bzw. Lösungspartialdruck des Sorbats im Substrat, je nachdem, ob das Substrat von einem Gas oder einer Flüssigkeit umgeben ist. Unter Berücksichtigung der ebenfalls gemessenen Temperatur des Substrats läßt sich von dem Dampf- bzw. Lösungspartialdruck des Sorbats im Substrat auf den Sorbatgehalt des Substrats zurückschließen.

Das erfindungsgemäße Verfahren weist den Vorteil auf, daß relativ hohe Sorbatgehalte, insbesondere Feuchtegehalte, von Substraten auf der Grundlage der Bestimmung des thermodynamischen Gleichgewichtszustandes, und somit bei der Kompostierung von biogenen Abfällen ohne Einbeziehung des biologisch nicht relevanten Wassers, gemessen werden können.

Somit ist das erfindungsgemäße Verfahren insbesondere bei der Bestimmung der relativ hohen Feuchtegehalte von Kompostanlagen von Vorteil. Die Beeinflussung der Substrattemperatur durch ein wärmeres Trägermedium in der Meßkammer wird dadurch gering gehalten werden, daß sich dieses nicht ständig auf einem hohen Temperaturniveau befindet.

Gemäß einer vorteilhaften Ausgestaltung der Erfindung hat die Temperatur des Trägermediums in der Meßkammer anfangs einen Wert, bei dem das Trägermedium mit dem Sorbat gesättigt ist. Durch eine anschließende Variation der Temperatur des Trägermediums, in der Regel eine Erhöhung der Temperatur, steigt der Sorbatpartialdruck des Trägermediums in der Meßkammer zunächst an. Dabei bleibt das Trägermedium weiterhin mit Sorbat gesättigt, bis die Temperatur des Trägermediums erreicht ist, bei der der Sättigungs-Sorbatpartialdruck des Trägermediums gleich dem Dampf- bzw. Lösungspartialdruck des Sorbats im Substrat ist. Bei einer weiteren Erhöhung der Temperatur nimmt der Sorbatpartialdruck des Trägermediums nicht mehr weiter zu, sondern bleibt konstant. Dieser konstante Sorbatpartialdruck des Trägermediums wird zur oben beschriebenen Ermittlung des Feuchtegehalts des Substrats herangezogen.

Es ist vorteilhaft, kontinuierlich die Temperatur des Trägermediums in der Meßkammer zu verändern und den zugehörigen Sorbatpartialdruck des Trägermediums zu ermitteln. So kann der Sorbatpartialdruck des Trägermediums beispielsweise protokolliert werden.

Der Sorbatpartialdruck des Trägermediums kann unmittelbar gemessen werden, er kann aber auch über eine in Abhängigkeit von der Temperatur des Trägermediums aufgenommene Kalibrierungskurve bestimmt werden.

Die Aufgabe wird ferner durch die Merkmale des Anspruchs 6 gelöst. Bei diesem erfindungsgemäßen Verfahren wird ebenfalls ein Sensor mit einer Meßkammer verwendet, die ein Trägermedium enthält, dessen Sorbatpartialdruck bestimmt wird, aus dem unter Berücksichtigung der gemessenen, im wesentlichen konstanten Temperatur des Sorbats der Sorbatgehalt des Substrats ermittelt wird. Bei im wesentlichen konstanter Temperatur des Trägermediums in der Meßkammer wird die Geschwindigkeit einer Annäherung des Sorbatpartialdrucks des Trägermediums an einen stabilen Endwert ermittelt. Da diese Annäherungsgeschwindigkeit von dem Dampf- bzw. Lösungsdruck des Sorbats im Substrat abhängt, kann von der Annäherungsgeschwindigkeit auf den Dampf- bzw. Lösungspartialdruck des Sorbats im Substrat zurückgeschlossen werden. Unter Berücksichtigung der ebenfalls gemessenen Temperatur des Substrats kann wiederum über den Dampf- bzw. Lösungspartialdruck des Sorbats im Substrat der gesuchte Sorbatgehalt des Substrats ermittelt werden.

Auch dieses erfindungsgemäße Verfahren weist den Vorteil auf, daß relativ hohe Feuchtegehalte von Substraten auf der Grundlage der Bestimmung der Gleichgewichtsfeuchte gemessen werden können.

Bei diesem erfindungsgemäßen Verfahren ist es zur Vereinfachung des Verfahrens sehr vorteilhaft, wenn die Oberfläche des Substrats so groß gewählt ist, daß sie nicht die Geschwindigkeit des Transports der Sorbatmoleküle in die Meßkammer und damit die Annäherungsgeschwindigkeit limitiert. Ob die Oberfläche des Substrats ausreichend groß ist, läßt sich auf der Grundlage von Messungen der Annäherungsgeschwindigkeit für mehrere Temperaturwerte des Trägermediums feststellen. Auch wenn dieses Verfahren bei einer Temperatur des Trägermediums angewendet werden kann, bei der das Trägermedium bei dem stabilen Endwert mit Sorbat gesättigt ist, ist es doch von Vorteil, die Temperatur des Trägermediums in der Meßkammer so einzustellen, daß das Trägermedium bei dem stabilen Endwert nicht gesättigt ist.

Gemäß einer vorteilhaften Ausgestaltung der Erfindung wird der Sorbatpartialdruck des Trägermediums bei beiden Verfahren mit Hilfe einer Messung der Absorption von Laserlicht durch die Sorbatmoleküle bestimmt. Vorteile der unten detaillierter beschriebenen Messung des Sorbatpartialdrucks mit Hilfe von Laserlicht liegen in der Genauigkeit und der Geschwindigkeit des Meßverfahrens.

Als Trägermedium eignet sich insbesondere Luft. Es sind aber auch andere Medien möglich, wie z. B. ein anderes Gas, eine Flüssigkeit oder ein Feststoff. Die Wahl eines geeigneten Trägermediums erfolgt unter dem Gesichtspunkt, ein günstiges Verhältnis der Abhängigkeit des relativen Sorbatgehalts im Trägermedium und des Sorbatgehalts im Substrat voneinander zu erhalten. Dieses Abhängigkeitsverhältnis wird als Sorptionsisotherme bezeichnet.

Im folgenden wird ein Sensor beschrieben, der zur Durchführung der erfindungsgemäßen Verfahren verwendet werden kann. Dieser Sensor enthält eine über eine Temperiereinheit temperierbare Meßkammer, die von einer wärmeisolierenden Schicht umgeben ist und mit Öffnungen in Form von Bohrungen, die im Verhältnis zu dem Volumen der Meßkammer klein sind, versehen ist. Beispielsweise sollte das Verhältnis der gesamten Querschnittsfläche aller Bohrungen zur Gesamtfläche der Meßkammerwandung 15 % nicht oder nicht wesentlich überschreiten. Ferner weist der Sensor mindestens einen Temperatursensor zur Messung der Temperatur in der Meßkammer, mindestens einen Temperatursensor zur Messung der Temperatur des Substrats sowie eine Meßeinrichtung zur Bestimmung des Sorbatpartialdrucks auf. Die wärmeisolierende Schicht kann beispielsweise eine Luftschicht sein, die sich zwischen einer inneren und einer äußeren Hülle der Meßkammer befindet. Ferner kann erfindungsgemäß vorgesehen sein, das Volumen der Meßkammer möglichst klein zu halten. Durch diese Ausgestaltung der Meßkammer und die wärmeisolierende Schicht wird erreicht, daß die verfahrensbedingt hervorgerufene Temperaturdifferenz zwischen dem Trägermedium in der Meßkammer und dem Substrat nur eine vernachlässigbar geringe treibende Kraft für die Einstellung eines physikalischen Gleichgewichtszustandes darstellt. Denn der prinzipiell stattfindende Austausch von Sorbatmolekülen des Trägermediums mit dem Substrat wird so gering gehalten, daß die Temperatur des Substrats nur geringfügig beeinflußt wird und der stationäre Zustand des Substrats über lange Zeit aufrechterhalten werden kann. Auf diese Weise wird das Substrat durch die Messung nicht nachweisbar beeinflußt.

Wenn der Sensor ausschließlich zur Durchführung des Verfahrens nach Anspruch 6 verwendet werden soll, ist der Temperatursensor zur Messung der Temperatur in der Meßkammer zwar wünschenswert, aber nicht zwingend erforderlich.

Wie oben beschrieben, ist es vorteilhaft, wenn der Sensor, sofern er ein durchstrahlbares Trägermedium enthält, eine Lasermeßstrecke mit einem Laser zur Durchstrahlung der Meßkammer und einem Detektor aufweist.

Zur Ansteuerung des Sensors ist eine Steuervorrichtung vorgesehen. Es kann vorgesehen sein, daß sie nicht nur zur elektrischen Versorgung des Sensors dient, sondern auch unter Verwendung bekannter Mittel so ausgelegt ist, daß sie einen automatischen Ablauf des jeweiligen Verfahrens ermöglicht

Im folgenden werden das erfindungsgemäße Verfahren gemäß Anspruch 1 und ein dafür erfindungsgemäß vorgesehener Sensor anhand von Ausführungsbeispielen näher erläutert Dabei wird auf die Zeichnungen Bezug genommen. Es zeigen:
Figur 1, die Aufnahmefähigkeit von Luft für Wassermoleküle in Abhängigkeit von der Temperatur;
Figur 2, die Aufnahme von Sorbat in der Meßkammer in Abhängigkeit von der Temperatur des Trägermediums in der Meßkammer;
Figur 3, eine schematische Skizze eines Aufbaus eines erfindungsgemäßen Sensors.

In Figur 1 ist die Aufnahmefähigkeit von Luft für Wasser in Abhängigkeit von der Temperatur dargestellt, wobei auf der Ordinate der Wasserdampfdruck in Pascal (Pa) aufgetragen ist. Der maximale Wasserdampfdruck nimmt mit ansteigender Temperatur ansteigend zu, was zur Folge hat, daß eine Sättigung der Luft mit Wassermolekülen um so später eintritt, je höher die Temperatur der Luft ist.

Diese Eigenschaft wird bei der nachfolgend beschriebenen Ausführungsform des Verfahrens gemäß Anspruch 1 ausgenutzt. Es wird zunächst eine relativ geringe Temperatur des Trägermediums in der Meßkammer eingestellt. Anschließend wird der Sensor in Kontakt mit dem zu messenden Substrat gebracht, so daß ein Austausch zwischen Sorbatmolekülen des Substrats, hier Wassermolekülen, und des Trägermediums stattfinden kann. Da anfangs der Wasserdampfpartialdruck des Substrats größer ist als der Wasserdampfpartialdruck des Trägermediums, findet zunächst aufgrund eines räumlichen Konzentrationsgefälles von Wassermolekülen ein gerichteter Transport von Wassermolekülen in das Trägermedium statt. Der Ausgleich ist abgeschlossen, wenn entweder die Wasserdampfpartialdrücke des Substrats und des Trägermediums gleich sind oder das Trägermedium gesättigt ist, d. h. das Trägermedium eine relative Luftfeuchte von 100 % erreicht hat. Dann diffundieren in beide Richtungen, nämlich aus dem Trägermedium heraus und in das Trägermedium hinein, gleich viele Wassermoleküle.

Im vorliegenden Beispiel ist die Temperatur des Trägermediums T₀ in der Meßkammer so gering gewählt, daß das Trägermedium gesättigt ist. Anschließend wird die Temperatur des Trägermediums in der Meßkammer erhöht, wobei eine weitere Nachlieferung von Wassermolekülen aus dem Substrat in das Trägermaterial stattfindet. Dabei bleibt das Trägermedium weiterhin gesättigt. Der zugehörige Verlaufsabschnitt des Wasserdampfpartialdrucks im Trägermedium ist in Figur 2 mit dem Bezugszeichen 1 bezeichnet. Der Verlaufsabschnitt 1 des Wasserdampfpartialdrucks ist linear und liegt stets bei 100% relative Feuchte des Trägermediums. Dieser Verlauf des Wasserdampfpartialdrucks ändert sich erst bei der Temperatur des Trägermediums, bei der der Sättigungsdampfdruck gleich dem Wasserdampfpartialdruck des Substrats ist. Diese Temperatur ist in Figur 2 mit T₁ bezeichnet. Bei einer weiteren Erhöhung der Temperatur des Trägermediums in der Meßkammer bleibt der Wasserdampfpartialdruck des Trägermediums konstant; er liegt dabei unterhalb des Sättigungs-Wasserdampfpartialdrucks des Trägermediums. Man erhält also für das Substrat den mit S₁ bezeichneten Verlauf des Wasserdampfpartialdrucks des Trägermediums.

Der mit p₁ bezeichnete Wert des Wasserdampfpartialdrucks des Tragermediums ist gleich dem Wasserdampfpartialdruck des Substrats. Dieser Wasserdampfpartialdruck des Substrats ist proportional der absoluten Feuchte des Substrats. Da der Proportionalitätsfaktor bekannt ist, kann aus dem Wasserdampfpartialdruck p₁ die gesuchte absolute Feuchte des Substrats bestimmt werden. Dabei bleibt das chemisch gebundene Wasser unberücksichtigt, da es aufgrund der festen Bindung nicht zum Wasserdampfpartialdruck des Substrats beiträgt.

In Figur 2 ist ein weiterer Verlauf S₂ des Wasserdampfpartialdrucks des Trägermediums dargestellt, der am gleichen Substrat, welches jedoch einen anderen Feuchtegehalt aufweist, gemessen wurde. Dieses Substrat weist einen höheren Wasserdampfpartialdruck als das erste Substrat auf, so daß das Trägermedium erst bei einer höheren Temperatur, nämlich der Temperatur T₂, aus dem Sättigungsbereich herausgelangt. Es wird somit für dieses Substrat der Wasserdampfpartialdruck p₂ gemessen.

Bei diesem Verfahren muß die Temperatur der Luft in der Meßkammer, also des Trägermediums, so lange erhöht werden, bis der Wasserdampfpartialdruck nicht mehr weiter ansteigt, das Trägermedium also nicht mehr gesättigt ist. Dabei wird als ausgezeichneter Wert des Wasserdampfpartialdrucks ab einer bestimmten Temperatur ein stabiler Wasserdampfpartialdruck erhalten. Während bei einer Sättigung des Trägermediums kein Rückschluß auf den Wasserdampfpartialdruck des Substrats möglich ist, ist der stabile Wert des Wasserdampfpartialdrucks des Trägermediums gleich dem Wasserdampfpartialdruck des Substrats und kann somit zur Bestimmung der absoluten Feuchte herangezogen werden.

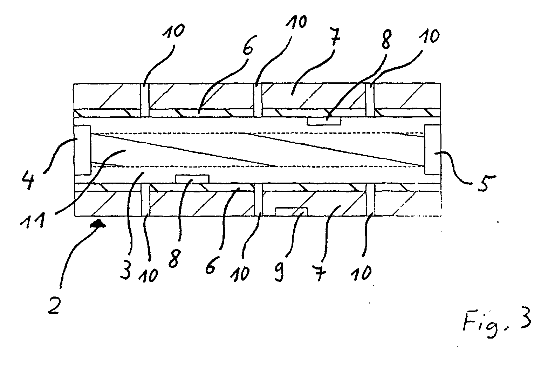

Der in Figur 3 dargestellte erfindungsgemäße Sensor 2 weist eine Meßkammer 3 auf, die ein Trägermedium (nicht gezeigt) enthält. Die Meßkammer 3 ist von einem wärmeleitenden Material umgeben. An einer der Stirnseiten der Meßkammer 3 ist eine Laserquelle 4 mit einer Fokussierung (nicht gezeigt) angebracht. An der gegenüberliegenden Stimseite der Meßkammer 3 befindet sich ein Detektor 5, der ebenfalls eine Fokussierung (nicht gezeigt) aufweist. Die Meßkammer 3 ist in Längsrichtung im wesentlichen von Temperierungselementen 6 in Form von Heizdrähten begrenzt. Diese Temperierungselemente 6 können aber auch Peltiereiemente sein. Zwischen den Temperierungselementen 6 und der Außenwand des Sensors 2 befinden sich wärmeisolierende Schichten 7, die durch mit Luft gefüllte abgeschlossene Kammern gebildet sind. Zwei Temperatursensoren 8 in Form von Platinwiderständen sind innerhalb der Meßkammer 3 an den Temperierungselementen 6 angebracht. Ein weiterer Temperatursensor 9 ist innerhalb einer der wärmeisolierenden Schichten 7 an der Außenwand des Sensors 2 angebracht. An jeder Längsseite des Sensors 2 befinden sich jeweils drei Bohrungen 10, die sich durch die wärmeisolierende Schicht 7 und das Temperierungselement 6 hindurcherstrecken. Die Bohrungen 10 ermöglichen einen Austausch des Sorbats, sind jedoch für das Substrat undurchlässig.

Die Laserquelle 4 und der Detektor 5 stellen eine Lasermeßstrecke 11 dar. Über die Temperierungselemente 6 und die Temperatursensoren 8 wird die Temperatur des Trägermediums der Meßkammer 3 mittels einer nicht gezeigten Steuervorrichtung eingestellt. Mit dem Temperatursensor 9, der an dem hier nicht gezeigten Substrat anliegt, kann die Temperatur des Substrats gemessen werden. Die wärmeisolierenden Schichten 7 sorgen dafür, daß eine möglichst geringe Wärmeübertragung von der Meßkammer 3 bzw. den Temperierungselementen 6 auf das Substrat stattfindet. Dadurch, daß die Meßkammer von wärmeleitendem Material umgeben ist, wird erreicht, daß in möglichst kurzer Zeit eine gleichmäßige Temperatur in der Meßkammer 3 geschaffen wird. Die Bohrungen 10 stellen Diffusionskanäle für den Austausch zwischen dem das Substrat umgebenden Transportmedium, durch welches die Sorbatmoleküle in die Meßkammer 3 transportiert werden, und dem Trägermedium dar. Die Bohrungen 10 weisen einen im Verhältnis zur Längsausdehnung der Meßkammer 3 kleinen Durchmesser auf. Mit Hilfe der Lasermeßstrecke 11 kann durch Absorptionsspektroskopie der Sorbatgehalt in dem Trägermedium bestimmt werden. Dabei wird das Trägermedium mit einem ersten Laserstrahl einer Wellenlänge eines Absorptionsmaximums der im Trägermedium enthaltenen Wassermoleküle durchstrahlt und die nicht absorbierte Laserstrahlung mit einem Detektor gemessen. Zusätzlich wird ein zweiter Laserstrahl einer Wellenlänge eingesetzt, die von den Wassermolekülen nicht absorbiert wird. Auch diese Laserstrahlung wird mit einem Detektor gemessen. Die Meßwerte werden aufgezeichnet, und aus der Differenz der Meßwerte wird der Wassergehalt des Trägermediums errechnet.

Der Sensor kann mittels einer Meßlanze in das Substrat eingebracht und so an beliebigen Stellen des Substrats zur Messung des Sorbatgehalts eingesetzt werden. Ebenso kann eine Ausführungsform der Meßlanze mehrere dieser Sensoren enthalten und damit die Messung des Sorbats in mehreren Ebenen des Substrats mit nur einer Meßlanze ermöglichen.

Ferner wird im folgenden das erfindungsgemäße Verfahren gemäß Anspruch 6 anhand eines Ausführungsbeispiels näher erläutert, bei dem ebenfalls der Wassergehalt eines Substrats zu messen ist.

Dazu wird der oben beschriebene Sensor 2 verwendet, wobei als Trägermedium wiederum Luft gewählt wird. Die Temperatur des Trägermediums in der Meßkammer wird so eingestellt, daß keine Sättigung des Trägermediums in der Meßkammer für den Zeitpunkt zu erwarten ist, zu dem der stabile Endwert des Wasserdampfpartialdrucks des Trägermediums vorliegt.

Der Sensor 2 wird in das Substrat eingeführt. Da im Substrat der Wasserdampfpartialdruck höher ist als im Trägermedium, findet auch hier wieder ein gerichteter Transport von Wassermolekülen in die Meßkammer 3 statt. Dabei ist die Geschwindigkeit der Annäherung des Wasserdampfpartialdrucks des Trägermediums an den stabilen Endwert, und damit die Geschwindigkeit des Ausgleichs des Wasserdampfpartialdrucks im Substrat und im Trägermedium, von der Größe des Unterschiedes des Wasserdampfpartialdrucks im Substrat und im Trägermedium abhängig. Der Ausgleich findet so lange statt, bis kein Druckunterschied mehr vorhanden ist, vorausgesetzt, daß keine Sättigung des Trägermediums in der Meßkammer mit Wasserdampf eintritt. Andernfalls findet kein weiterer Druckausgleich statt.

Während des Ausgleichs wird ständig der Wasserdampfpartialdruck durch die Laser-Meßstrecke 11 in Abhängigkeit von der gemessenen Zeit aufgezeichnet. Die Annäherungsgeschwindigkeit ergibt sich aus der ersten Ableitung der Änderung des Wasserdampfpartialdrucks des Trägermediums über die Zeit, was graphisch also dem Anstieg der aufgezeichneten Meßkurve entspricht. Damit ist aus der Messung mindestens zweier Werte des Wasserdampfpartialdrucks des Trägermediums unter Beachtung des zeitlichen Abstandes der Meßwerte während des Ausgleichsvorganges der Rückschluß auf den Wassergehalt im Substrat möglich.

Es kann generell bei dem Verfahren vorkommen, daß die Annäherungsgeschwindigkeit durch Randbedingungen begrenzt wird, so daß sie ihren möglichen Maximalwert nicht erreicht. Limitierende Bedingungen können eine kleine Oberfläche des Substrats oder auch eine geringe Molekülbeweglichkeit des Sorbats im Trägermedium sein. Grundsätzlich sollte daher möglichst darauf geachtet werden, daß die Oberfläche des Substrats so groß ist, daß sie nicht die Annäherungsgeschwindigkeit an den stabilen Endwert des Sorbatpartialdrucks des Trägermediums limitiert.

Eine Abschätzung dieser Randbedingungen im Betrieb ist nicht ohne weiteres möglich. Bei einer Einstellung der Temperatur des Trägermediums auf einen Wert, so daß während des Ausgleichvorganges keine Sättigung des Trägermediums in der Meßkammer auftritt, sind alle Phasen des Ausgleichs aufgezeichnet, und eine Überprüfung des Ausgleichsvorganges auf limitierende Randbedingungen wird möglich. Bei ganz geringem Sorbatpartialdruck-Unterschied ist die Annäherungsgeschwindigkeit sehr klein, und es kann dann von unlimitiertem Ausgleich ausgegangen werden.

Die Kalibrierung der Meßwerte des Sorbatpartialdrucks in der Meßkammer 3 erfolgt wie in dem Verfahren gemäß Anspruch 1 durch die Sorptionsisotherme.

Wenngleich die Erfindung in bezug auf die Messung von Wasser als Sorbat erläutert wurde, versteht sich jedoch, daß im Rahmen der Erfindung auch andere Sorbate als Wasser gemessen werden können.

## Patentansprüche

1. Verfahren zum Messen von Sorbatgehalten in Substraten, insbesondere von Wassergehalten in Kompostanlagen, bei dem ein Sensor mit einer Meßkammer verwendet wird, die ein Trägermedium enthält, dessen Sorbatpartialdruck bestimmt wird, aus dem unter Berücksichtigung der gemessenen, im wesentlichen konstanten Temperatur des Substrats der Sorbatgehalt des Substrats ermittelt wird, wobei die Temperatur des Trägermediums in der Meßkammer bis zu einem Wert und etwas über diesen hinaus variiert wird, bei welchem sich der Sorbatpartialdruck in der Meßkammer nicht mehr in Abhängigkeit von der Temperatur des Trägermediums ändert, und dieser Sorbatpartialdruck zur Ermittlung des Sorbatgehalts des Substrats herangezogen wird.

2. Verfahren nach Anspruch 1, **dadurch gekennzeichnet, daß** die Temperatur des Trägermediums in der Meßkammer von einem Wert ausgehend, bei dem das Trägermedium mit dem Sorbat gesättigt ist, erhöht wird.

3. Verfahren nach Anspruch 1 oder 2, **dadurch gekennzeichnet, daß** kontinuierlich die Temperatur des Trägermediums in der Meßkammer verändert und der jeweils zugehörige Sorbatpartialdruck des Trägermediums ermittelt wird.

4. Verfahren nach einem oder mehreren der vorhergehenden Ansprüche, **dadurch gekennzeichnet, daß** der Sorbatpartialdruck des Trägermediums unmittelbar gemessen wird.

5. Verfahren nach einem oder mehreren der vorhergehenden Ansprüche, **dadurch gekennzeichnet, daß** der Sorbatpartialdruck des Trägermediums über eine in Abhängigkeit von der Temperatur des Trägermediums aufgenommene Kalibrierungskurve bestimmt wird.

6. Verfahren zum Messen von Sorbatgehalten in Substraten, insbesondere von Wassergehalten in Kompostanlagen, bei dem ein Sensor mit einer Meßkammer verwendet wird, die ein Trägermedium enthält, dessen Sorbatpartialdruck bestimmt wird, aus dem unter Berücksichtigung der gemessenen, im wesentlichen konstanten Temperatur des Substrats der Sorbatgehalt des Substrats ermittelt wird, wobei bei im wesentlichen konstanter Temperatur des Trägermediums in der Meßkammer die Geschwindigkeit der Annäherung des Sorbatpartialdrucks des Trägermediums an den stabilen Endwert ermittelt wird und unter Berücksichtigung des bekannten Abhängigkeitsverhältnisses der Annäherungsgeschwindigkeit und des Dampf- bzw. Lösungspartialdrucks des Sorbats im Substrat der Sorbatgehalt des Substrats ermittelt wird.

7. Verfahren nach Anspruch 6, **dadurch gekennzeichnet, daß** die Oberfläche des Substrats so groß gewählt ist, daß sie nicht die Annäherungsgeschwindigkeit limitiert.

8. Verfahren nach Anspruch 6 oder 7, **dadurch gekennzeichnet, daß** die Temperatur des Trägermediums in der Meßkammer so eingestellt wird, daß es bei dem stabilen Endwert nicht gesättigt ist.

9. Verfahren nach einem der vorhergehenden Ansprüche, **dadurch gekennzeichnet, daß** der Sorbatpartialdruck des Trägermediums mit Hilfe einer Messung der Absorption von Laserlicht durch die Sorbatmoleküle bestimmt wird.

10. Verfahren nach einem der vorhergehenden Ansprüche, **dadurch gekennzeichnet, daß** das Trägermedium Luft ist.

11. Sensor zur Durchführung des Verfahrens nach Anspruch 1 oder Anspruch 6, **gekennzeichnet durch** eine über eine Temperiereinheit (6) temperierbare Meßkammer (3), die von einer wärmeisolierenden Schicht (7) umgeben und mit Öffnungen in Form von Bohrungen (10), die im Verhältnis zu dem Volumen der Meßkammer (3) klein sind, versehen ist, sowie **durch** mindestens einen Temperatursensor (8) zur Messung der Temperatur in der Meßkammer (3), mindestens einen Temperatursensor (9) zur Messung der Temperatur des Substrats und eine Meßeinrichtung (4, 5, 11) zur Bestimmung des Sorbatpartialdruckes.

12. Sensor zur Durchführung des Verfahrens nach Anspruch 6, **gekennzeichnet durch** eine über eine Temperiereinheit (6) temperierbare Meßkammer (3), die von einer wärmeisolierenden Schicht (7) umgeben und mit Öffnungen in Form von Bohrungen (10), die im Verhältnis zu dem Volumen der Meßkammer (3) klein sind, versehen ist, sowie **durch** mindestens einen Temperatursensor (9) zur Messung der Temperatur des Substrats und eine Meßeinrichtung (4, 5, 11) zur Bestimmung des Sorbatpartialdruckes.

13. Sensor nach Anspruch 11 oder 12, **dadurch gekennzeichnet, daß** die Meßeinrichtung zur Bestimmung des Sorbatpartialdruckes eine Lasermeßstrecke (11) mit einer Laserquelle (4) zur Durchstrahlung der Meßkammer (3) und einem Detektor (5) aufweist.

## Claims

1. Method of measuring sorbate contents in substrates, in particular water contents in composting plants, in which a sensor with a measuring chamber containing a carrier medium is used, of which the sorbate partial pressure is determined, from which the sorbate content of the substrate is determined, while taking into account the measured, substantially constant temperature of the substrate, wherein the temperature of the carrier medium in the measuring chamber varies up to a value, and slightly beyond this, at which the sorbate partial pressure in the measuring chamber no longer changes as a function of the temperature of the carrier medium and this sorbate partial pressure is used to determine the sorbate content of the substrate.

2. Method according to claim 1, **characterised in that** the temperature of the carrier medium in the measuring chamber is increased, starting from a value at which the carrier medium is saturated with the sorbate.

3. Method according to claim 1 or 2, **characterised in that** the temperature of the carrier medium in the measuring chamber is continuously changed and the associated respective sorbate partial pressure of the carrier medium is determined.

4. Method according to any one or more of the preceding claims, **characterised in that** the sorbate partial pressure of the carrier medium is measured directly.

5. Method according to any one or more of the preceding claims, **characterised in that** the sorbate partial pressure of the carrier medium is determined over a calibration curve drawn up as a function of the temperature of the carrier medium.

6. Method of measuring sorbate contents in substrates, in particular water contents in composting plants, in which a sensor with a measuring chamber containing a carrier medium is used, of which the sorbate partial pressure is determined from which the sorbate content of the substrate is determined, while taking into account the measured, substantially constant temperature of the substrate, wherein the speed at which the sorbate partial pressure of the carrier medium approaches the steady final value is determined at a substantially constant temperature of the carrier medium in the measuring chamber and the sorbate content of the substrate is determined while taking into account the known dependent relationship of the approach speed and of the vapour or solution partial pressure of the sorbate in the substrate.

7. Method according to claim 6, **characterised in that** the surface of the substrate is selected so large that it does not limit the approach speed.

8. Method according to claim 6 or 7, **characterised in that** the temperature of the carrier medium in the measuring chamber is adjusted such that it is not saturated at the steady final value.

9. Method according to any of the preceding claims, **characterised in that** the sorbate partial pressure of the carrier medium is determined by measurement of the absorption of laser light by the sorbate molecules.

10. Method according to any of the preceding claims, **characterised in that** the carrier medium is air.

11. Sensor for carrying out the method according to claim 1 or claim 6, **characterised by** a measuring chamber (3) which can be temperature-controlled via a temperature-control unit (6) and is surrounded by a heat-insulating layer (7) and provided with apertures in the form of holes (10) which are small in relation to the volume of the measuring chamber (3), and by at least one temperature sensor (8) for measuring the temperature in the measuring chamber (3), at least one temperature sensor (9) for measuring the temperature of the substrate and a measuring device (4, 5, 11) for determining the sorbate partial pressure.

12. 'Sensor for carrying out the method according to claim 6, **characterised by** a measuring chamber (3) which can be temperature-controlled via a temperature-control unit (6) and is surrounded by a heat-insulating layer (7) and provided with apertures in the form of holes (10) which are small in relation to the volume of the measuring chamber (3), and by at least one temperature sensor (9) for measuring the temperature of the substrate and a measuring device (4, 5, 11) for determining the sorbate partial pressure.

13. Sensor according to claim 11 or 12, **characterised in that** the measuring device for determining the sorbate partial pressure comprises a laser measuring section (11) with a laser source (4) for irradiating the measuring chamber (3), and a detector (5).

## Revendications

1. Procédé de mesure des teneurs en sorbats dans des substrats, notamment des teneurs en eau dans des composts, procédé dans lequel on utilise un capteur ayant une chambre de mesure, qui contient un milieu porteur, dont est définie la pression partielle du sorbat, et à partir de laquelle la teneur en sorbat du substrat est déterminée en tenant compte de la température mesurée, sensiblement constante, du substrat, la température du milieu porteur présent dans la chambre de mesure étant amenée à varier jusqu'à une valeur et un peu au-delà de celle-ci, stade auquel la pression partielle du sorbat dans la chambre de mesure ne se modifie plus en fonction de la température du milieu porteur, et c'est cette pression partielle du sorbat qui est utilisée pour la détermination de la teneur en sorbat du substrat.

2. Procédé selon la revendication 1, **caractérisé en ce que** la température du milieu porteur dans la chambre de mesure est augmentée à partir d'une valeur à laquelle le milieu porteur est saturé de sorbat.

3. Procédé selon la revendication 1 ou 2, **caractérisé en ce que** la température du milieu porteur, présent dans la chambre de mesure, est modifiée en continu et **en ce qu'**est déterminée la pression partielle du sorbat, chaque fois correspondante, du milieu porteur.

4. Procédé selon une ou plusieurs des revendications précédentes, **caractérisé en ce que** la pression partielle du sorbat du milieu porteur est mesurée directement.

5. Procédé selon une ou plusieurs des revendications précédentes, **caractérisé en ce que** la pression partielle du sorbat du milieu porteur est déterminée par l'intermédiaire d'une courbe d'étalonnage, établie en fonction de la température du milieu porteur.

6. Procédé de mesure des teneurs en sorbats dans des substrats, notamment des teneurs en eau dans des composts, procédé dans lequel on utilise un capteur ayant une chambre de mesure, qui contient un milieu porteur, dont est définie la pression partielle du sorbat, et à partir de laquelle la teneur en sorbat du substrat est déterminée en tenant compte de la température mesurée, sensiblement constante, du substrat, et dans lequel, pour une température sensiblement constante du milieu porteur présent dans la chambre de mesure, est mesurée la vitesse à laquelle la pression partielle du sorbat du milieu porteur se rapproche de la valeur finale stable, la teneur en sorbat du substrat étant déterminée en tenant compte du rapport connu de dépendance de la vitesse de rapprochement et de la pression partielle de la vapeur ou de la solution du sorbat dans le substrat.

7. Procédé selon la revendication 6, **caractérisé en ce que** la surface du substrat est choisie suffisamment grande pour ne pas limiter la vitesse de rapprochement.

8. Procédé selon la revendication 6 ou 7, **caractérisé en ce que** la température du milieu porteur, présent dans la chambre de mesure, est réglée de telle manière que ce milieu ne soit pas saturé, à la valeur finale stable.

9. Procédé selon l'une des revendications précédentes, **caractérisé en ce que** la pression partielle du sorbat du milieu porteur est déterminée à l'aide d'une mesure de l'absorption d'une lumière laser par les molécules du sorbat.

10. Procédé selon l'une des revendications précédentes, **caractérisé en ce que** le milieu porteur est l'air.

11. Capteur destiné à la mise en oeuvre du procédé selon la revendication 1 ou la revendication 6, **caractérisé par** une chambre de mesure (3), propre à être tempérée par l'intermédiaire d'une unité d'équilibrage de température (6), cette chambre étant entourée par une couche d'isolation thermique (7) et étant pourvue d'ouvertures sous la forme d'alésages (10), qui sont petits par rapport au volume de la chambre de mesure (3), ainsi que par au moins un capteur de température (8) prévu pour mesurer la température régnant dans la chambre de mesure (3), au moins un capteur de température (9) servant à la mesure de la température du substrat, et un dispositif de mesure (4, 5, 11) pour la détermination de la pression partielle du sorbat.

12. Capteur destiné à la mise en oeuvre du procédé selon la revendication 6, **caractérisé par** une chambre de mesure (3), propre à être tempérée par l'intermédiaire d'une unité d'équilibrage de température (6), cette chambre étant entourée par une couche d'isolation thermique (7) et étant pourvue d'ouvertures sous la forme d'alésages (10), qui sont petits par rapport au volume de la chambre de mesure (3), ainsi que par au moins un capteur de température (9) prévu pour mesurer la température du substrat, et un dispositif de mesure (4, 5, 11) pour la détermination de la pression partielle du sorbat.

13. Capteur selon la revendication 11 ou 12, **caractérisé en ce que** le dispositif de mesure, servant à la détermination de la pression partielle du sorbat, présente une section de mesure par laser (11), avec une source laser (4) pour l'émission d'un rayonnement à travers la chambre de mesure (3), et un détecteur (5).
